(19) Europäisches Patentamt
European Patent Office
Office européen des brevets

(11) Veröffentlichungsnummer : **0 009 077**
**B1**

(12) **EUROPÄISCHE PATENTSCHRIFT**

(45) Veröffentlichungstag der Patentschrift : **30.09.81**

(51) Int. Cl.$^3$ : **C 07 C 21/24**, C 07 C 17/26, C 07 C 25/02, C 07 C 43/225, C 07 C 49/80, C 07 C 79/12

(21) Anmeldenummer : **79102258.5**

(22) Anmeldetag : **04.07.79**

(54) **Substituierte Phenylpropylhalogenide, ihre Herstellung und Verwendung.**

(30) Priorität : **08.07.78 DE 2830120**

(43) Veröffentlichungstag der Anmeldung : **02.04.80 (Patentblatt 80/07)**

(45) Bekanntmachung des Hinweises auf die Patenterteilung : **30.09.81 Patentblatt 81/39**

(84) Benannte Vertragsstaaten : **AT BE CH DE FR GB IT LU NL SE**

(56) Entgegenhaltungen :

**DE - A1 - 2 752 135**
**FR - A1 - 2 364 194**
**US - A - 3 052 733**
**US - A - 4 051 190**

**Chemical Abstracts Band 45, nr. 49, 10. Oktober 1951 (COLUMBUS, OHIO, USA)**
**R. TRUFFAULT et al. « Alkylation of halogen substituted aromatic hydrocarbons with ethylenes in presence of sulfuric acid as catalyst » Spalte 8494 D-G**

(73) Patentinhaber : **BASF Aktiengesellschaft**
**Carl-Bosch-Strasse 38**
**D-6700 Ludwigshafen (DE)**

(72) Erfinder : **Buschmann, Ernst, Dr.-Chem.**
**An der Froschlache 7**
**D-6700 Ludwigshafen (DE)**
Erfinder : **Goetz, Norbert, Dr.-Chem.**
**Schoefferstrasse 25**
**D-6520 Worms (DE)**
Erfinder : **Zeeh, Bernd, Dr.-Chem.**
**Thorwaldsenstrasse 5**
**D-6700 Ludwigshafen (DE)**
Erfinder : **Varwig, Juergen, Dr.-Chem.**
**Am Goetzenberg 1**
**D-6900 Heidelberg (DE)**

**EP 0 009 077 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

# Substituierte Phenylpropylhalogenide, ihre Herstellung und Verwendung

Die vorliegende Erfindung betrifft ein Verfahren zur Herstellung von substituierten Phenylpropylhalogeniden durch Umsetzung von Phenylpropylhalogeniden mit Alkoholen, Alkylhalogeniden oder Olefinen in Gegenwart saurer Katalysatoren und die so erhältlichen Produkte. Sie sind wichtige Ausgangsverbindungen für die Herstellung von entsprechend substituierten Morpholinen oder Piperidinen, die zur Bekämpfung von Pilzen verwendet werden können. Es ist bekannt, Alkylaromaten aus Aromaten und Olefinen mit stark sauren Katalysatoren wie z.b. Schwefelsäure oder Phosphorsäure herzustellen (Friedel-Crafts and Related Reactions, George A. Olah Band II, S. 1-288, 1964).

Beispielsweise liefert die Alkylierung von Toluol mit Isobutylen in sehr guter Ausbeute p-tert.-Butyltoluol. Setzt man statt Toluol Aromaten mit längeren Alkylketten (z.B. Äthylbenzol, Propylbenzol) ein, so verläuft die Umsetzung deutlich langsamer (R. N. Volkor u. S. V. Zavgorodnij, Dokl. Akad. Nauk S.S.S.R. 133, 843 (1960)). Die Alkylierung von Aromaten mit einer labilen halogenierten Seitenkette unter den üblichen Bedingungen ist nur aus einem Beispiel bekannt (Y. Monteils, Bull. Soc. Chim. France 636, 1951), wo sie eine unbefriedigende Ausbeute ergibt.

Es ist bekannt, substituierte Phenylpropylmorpholin- oder -piperidinverbindungen als Fungizide zu verwenden (DE-OS 27 52 135). Sie werden hergestellt durch Umsetzung von substituierten Phenylpropylhalogeniden mit substituierten Morpholinen oder Piperidinen. Es ist ferner bekannt, aromatische substituierte Alkylhalogenide durch Umsetzung von Halogenalkylen mit Halogen-aromaten in Gegenwart von Aluminiumchlorid herzustellen (FR-PS 23 64 194). Aus C. A. 45, 8494 (1951) ist es bekannt, Cyclohexen mit Chlorphenylen in Gegenwart von Schwefelsäure zu halogensubstituierten Cyclohexenaromatischen Kohlenwasserstoffen umzusetzen.

Aus der US-PS 4 051 190 ist die Herstellung von 3-Arylpropylchloriden aus 2-Chlorpropiophenonen durch Hydrierung bekannt. Aus US-PS 3 052 733 ist die Herstellung von Beta-haloalkyl-alkylarylverbindungen aus Beta-haloalkyl-arylverbindungen und Olefinen in Gegenwart von Fluorwasserstoff oder Titanfluorid bekannt.

Es wurde nun gefunden, daß man Phenylpropylhalogenide der allgemeinen Formel

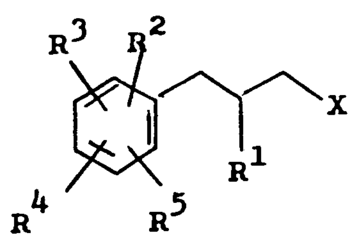

(I)

in der $R^1$ Wasserstoff oder einen aliphatischen Rest mit 1 bis 10 Kohlenstoffatomen.
$R^2$ Wasserstoff, einen Alkylrest mit 1 bis 3 Kohlenstoffatomen, Fluor oder einen Methoxyrest,
$R^3$ einen aliphatischen Rest mit 2 bis 20 Kohlenstoffatomen, einen cyclo- oder bicycloaliphatischen Rest mit 5 bis 7 Kohlenstoffatomen oder einen Halogenalkylrest mit 3 bis 10 Kohlenstoffatomen,
$R^4$ und $R^5$ jeweils unabhängig voneinander Wasserstoff, Chlor, Brom, Nitro oder Acetyl und X Chlor oder Brom bedeuten, in einfacher Weise und guten Ausbeuten erhält, wenn man ein Phenylhalogenid der Formel

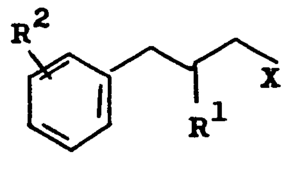

(II)

worin $R^1$, $R^2$ und X die oben angegebenen Bedeutungen haben in Gegenwart eines sauren Katalysators mit einem Alkohol der Formel $R^3OH$ oder einem Alkylhalogenid der Formel $R^3Hal$, wobei Hal ein Halogenatom bedeutet, oder einem der Verbindung $R^3H$ entsprechenden Olefin umsetzt, wobei $R^3$ die oben genannten Bedeutungen hat und gegebenenfalls das so erhaltene Umsetzungsprodukt zu dem entsprechenden Halogenderivat, Nitroderivat oder Acetylderivat umsetzt.

Die nach diesem Verfahren erhältlichen substituierten Phenylpropylhalogenide können beispielsweise mit gegebenenfalls substituierten Morpholinen oder Piperidinen zu Verbindungen umgesetzt werden, die eine gute fungizide Wirkung haben und als Fungizide zur Bekämpfung von Pilzen verwendet werden Können (DE-OS 2 656 747, 2 752 135).

Insbesondere die substituierten Phenylpropylhalogenide der allgemeinen Formel

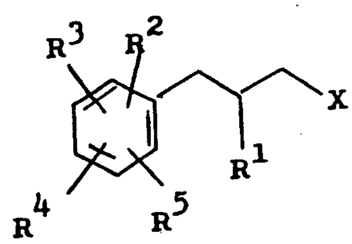

(III)

in der $R^1$ Wasserstoff oder einen aliphatischen Rest mit 1 bis 10 Kohlenstoffatomen, $R^2$ Wasserstoff, einen Alkylrest mit 1 bis 3 Kohlenstoffatomen, Fluor oder einem Methoxyrest, $R^3$ einen aliphatischen Rest mit 2 bis 20 Kohlenstoffatomen, einen cyclo- oder bicycloaliphatischen Rest mit 5 bis 7 Kohlenstoffatomen oder einen Halogenalkylrest mit 3 bis 10 Kohlenstoffatomen, $R^4$ und $R^5$ jeweils unabhängig voneinander Wasserstoff, Chlor, Brom, Nitro oder Acetyl und X Chlor oder Brom bedeutet, mit der Maßgabe, daß $R^3$ einen Halogenalkylrest mit 3 bis 10 Kohlenstoffatomen bedeutet, wenn $R^2$, $R^4$ und $R^5$ gleichzeitig Wasserstoff bedeuten, sind für die Herstellung von fungiziden Wirkstoffen entsprechend der oben beschriebenen Umsetzung gut geeignet.

Beispielsweise erhält man durch Umsetzung von 3-[p-(1-Chlormethyl-1-methyl-ethyl)-phenyl]-2-methyl-propylchlorid mit cis-2,6-Dimethylmorpholin das N-[3-(p-1-Chlormethyl-1-methyl-ethyl-phenyl)-2-methyl]-propyl-2,6-cisdimethylmorpholin entsprechend der folgenden Arbeitsweise : Eine Mischung von 24 g 3-p-(1-Chlormethyl-1-methyl-ethyl-phenyl)-2-methyl-propylchlorid und 32 g cis-2,6-Dimethylmorpholin wird 4 Stunden auf 150 °C erhitzt. Das Rohprodukt wird in Chloroform gelöst, die Lösung mehrfach mit Wasser gewaschen, über $Na_2SO_4$ getrocknet, das Lösungsmittel abgedampft und der Rückstand destilliert. Man erhält so 21 g N-[3-(p-1-Chlormethyl-1-methyl-ethyl-phenyl)-2-methyl]-propyl-2,6-cis-dimethyl-morpholin, $Kp_{0,1}$ 165-166 °C.

$R^1$ bedeutet beispielsweise Wasserstoff, einen Methyl-, Ethyl-, Propyl-, Isopropyl-, 1-Methyl-propyl, 2-Methyl-propyl-, Butyl, Pentyl- oder Hexylrest. $R^2$ bedeutet beispielsweise Wasserstoff, Fluor, einen Methyl-, Äthyl-, Propyl- oder einen Methoxyrest.

$R^3$ bedeutet beispielsweise einen tert.-Butyl-, tert.-Amyl-, 1-Ethyl-1-methyl-propyl-, 1,1-Diethyl-propyl-, 1,1-Dimethyl-butyl-, 1,1-Dimethyl-pentyl-, Cyclopentyl-, Cyclohexyl-, Cycloheptyl-, Norbornyl-, 1-Chlormethyl-1-methyl-ethyl- oder einen 3-Chlor-1,1-dimethyl-propylrest.

Als Ausgangsverbindungen für das verfahren dienen Phenylpropylhalogenide der Formel

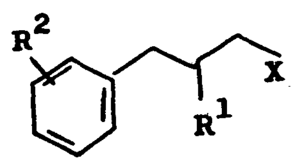

in der $R^1$, $R^2$ und X die oben genannten Bedeutungen haben, die nach folgendem Schema nach allgemein bekannten Reaktionen zugänglich sind :

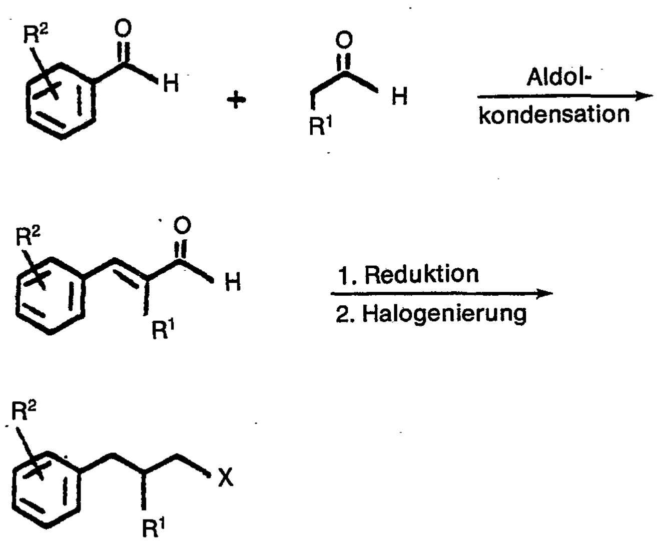

(siehe u. a. G. Baddeley, R. Williamson, J. Chem. Soc. *1956*, 4651).

Weitere Ausgangsstoffe sind Olefine, die der Verbindung $R^3H$ entsprechen, wobei $R^3$ die oben genannten Bedeutungen hat. Dies sind beispielsweise : Äthylen, n-Penten-(1), n-Hexen-(1), n-Hepten-(1), n-Octen-(1), n-Nonen-(1), n-Decen-(1), n-Undecen-(1), n-Dodecen-(1), Propylen-(1), n-Buten-(1) ; vorgenannte Alkene, die in 2-Stellung oder 3-Stellung oder 4-Stellung durch die Methyl-, Äthyl-, n-Propyl-, Isopropyl-, n-Butyl-, Isobutyl-, sek.-Butyl-, tert.-Butyl-Gruppe substituiert sind ; 2,3-Dimethyl-n-buten, 3,3-Dimethyl-n-buten, 2,5-Dimethylhepten, 3,3-Dimethylhepten, 2,3,4-Trimethylhepten, 2,4-Dimethylhepten, 2,3-Dimethylhepten, 4,4-Dimethylhepten, 2,3-Diäthylhexen, 4,4-Dimethylhexen, 2,3-Dimethylhexen, 2,4-Dimethylhexen, 2,5-Dimethylhexen, 3,3-Dimethylhexen, 3,4-Dimethylhexen, 2-Methyl-3-äthylpenten, 3-Methyl-3-äthylpenten, 2,3,3-Trimethylhepten, 2,4,4-Trimethylpenten, 2,3,3,-Trimethylpenten, 2,3,4-Trimethylpenten, 2,3,3,4,-Tetramethylpenten ; analoge Alkene, deren Doppelbindung in 2-Stellung oder in 3-Stellung im Molekül liegen ; verzweigte Alkene, wie sie in Gestalt von Gemischen bei der Dimerisierung von Isobutylen oder n-Buten (Octene) oder Trimerisierung von Isobutylen oder n-Buten (Dodecene) oder Propylen (Nonene) bzw. Tetramerisierung von Propylen (Dodecene) anfallen ; die cyclischen und bicyclischen Olefine : Cyclohepten, Cyclohexen, Cyclopenten, Norbornen sowie die halogenierten Olefine : Vinylbromid, Allylchlorid, Allylbromid, 1,3-Dichlorpropen, Methylallylchlorid, Crotylchlorid, 1,3-Dichloro-2-methyl-1-propen, 2-Methyl-allylchlorid, 2-Chlormethyl-allyl-chlorid und 4-Chlor-2-methyl-buten-1.

Bevorzugt werden : Norbornen, Cyclopenten, Cyclohexen, Cyclohepten, Isobutylen, 1-Hepten und Methallylchlorid.

Alternativ können Halogenide der Formel $R^3Hal$ als Ausgangsstoffe verwendet werden, beispielsweise die folgenden Verbindungen : Äthylbromid, n-Propylchlorid, n-Propylbromid, Isopropylchlorid, Isopropylbromid, n-Butylchlorid, s-Butylchlorid, Isobutylchlorid, t-Butylchlorid, Isoamylchlorid, t-Amylchlorid, t-Amylbromid, Neopentylchlorid, 1,1-Diethyl-ethylchlorid, 1,1-Diethyl-propylchlorid, 1,1-Dimethyl-butylchlorid, 1,1-Dimethylpentylchlorid, 1,3-Dichlor-1,1-dimidethylpropan, Cyclopentylchlorid, 2-Chloro-2-methylpentan, 3-Chloro-3-methylpentan, Cyclohexylchlorid, 3-Chloro-2-methylhexan, 3-Chloro-3-äthylpentan, 2,4-Dimethyl-2-chloropentan, 3-Methylcyclohexylchlorid, 4-Chloro-4-methylheptan, 2-Chloro-2,5-dimethylhexan, 3-Chloro-3-äthylhexan, 4-Chloro-4-äthylheptan, 3-Chloro-3,6-dimethylheptan, 3-Chloro-3-äthyl-5-methylhexan, 4-Chloro-4n-propylheptan, 4-Chloro-2,4,6-trimethylheptan, Methylchlorid, 4-Chloro-4n-propyl-2-methylheptan, 5-Phenyl-1-chloropentan, 5-Chloro-2,5,8-trimethylnonan, 5-Chloro-2,8-dimethyl-5-äthylnonan, 5-Chloro-2,8-dimethyl-5n-propylnonan, 5-Chloro-2,8-dimethyl-5-isobutylnonan, n-Octadecylbromid, 1,3-Dichlor-1,1-dimethylpropan und 1,2,3-Trichlor-1,1-dimethylpropan. Bevorzugt werden : tert.-Butylchlorid, tert.-Amylchlorid, 1,1-Diethyl-ethylchlorid, 1,1-Diethyl-propylchlorid, 1,1-Dimethylbutylchlorid, 1,1-Dimethyl-pentylchlorid und 1,3-Dichlor-1,1-dimethylpropan.

Alternativ können Alkohole der Formel $R^3OH$, beispielsweise die folgenden Alkohole verwendet werden : Methanol, Äthanol, n-Propylalkohol, Isopropylalkohol, n-Butanol, s-Butanol, Isobutanol, t-Butanol, n-Amylalkohol, 2-Pentanol, 3-Methyl-2-butanol, t-Amylalkohol, Neopentylalkohol, Cyclobutylcarbinol, 2-Methyl-2-pentanol, 2,3-Dimethyl-2-butanol, Cyclohexanol, Cyclopentylcarbinol; Cyclohexylcarbinol, n-Octylalkohol, 2-Methyl-2-heptanol, 2,3-Dimethyl-2-hexanol, 2,4-Dimethyl-2-hexanol, 3-Äthyl-2-methyl-2-pentanol, 2,3,3-Trimethyl-2-pentanol, 2,4,4-Trimethyl-2-pentanol, α-Phenylpropanol, α-Dodecylalkohol. Bevorzugt werden : tert.-Butanol und t-Amylalkohol.

Als saure Katalysatoren können beispielsweise verwendet werden : $AlCl_3$, $AlBr_3$, $FeCl_3$, $TiCl_4$, $ZrCl_4$, $VCl_4$, $ZnCl_2$, $ZnCl_2.Al_2O_3$, $ZnCl_2.SiO_2$, $BF_3$, $BF_3.2H_2O$, $BF_3.H_2SO_4$, $BF_3.H_3PO_4$, HBr, $H_2SO_4$, HF, $FSO_3H$, HF, $FSO_3H$, $P_2O_5$, Polyphosphorsäure, $SiO_2.Al_2O_3$, $CH_3SO_3H$ oder Homologe, $ClSO_3H$, $HClO_4$, Toluolsulfonsäure, organische sulfonsaure Kationenaustauscher.

Bevorzugt werden : HF, $H_2SO_4$, $FeCl_3$ und $BF_3.2H_2O$.

Der saure Katalysator liegt bei der Umsetzung beispielsweise in einer Menge von 5 bis 100 Gewichtsprozenten bezogen auf das Ausgangsprodukt Phenylpropylhalogenid vor.

Die Umsetzung verläuft beispielsweise bei einer Temperatur von − 10° bis 250 °C, vorzugsweise bei 5-50 °C, bei Normaldruck oder erhöhtem Druck. Die Reaktion läuft nach folgender Gleichung ab :

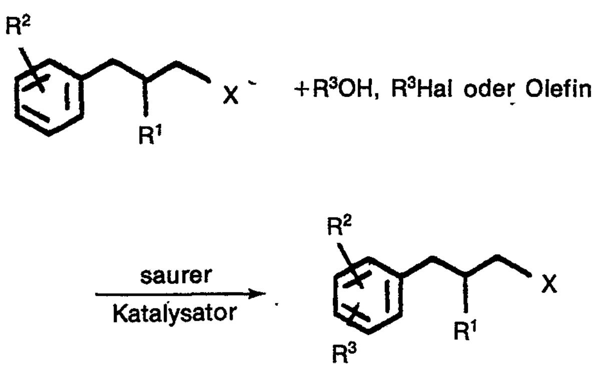

Die Umsetzung kann beispielsweise wie folgt durchgeführt werden : Zum Gemisch aus Katalysator und Halogenid, gegebenenfalls zusammen mit einem Lösungsmittel, wird das Alkylierungsmittel zugegeben. Nach Ende der Umsetzung wird das Rohprodukt nach Abtrennung des Katalysators gegebenenfalls in einem organischen Lösungsmittel aufgenommen, mit Wasser gewaschen und destilliert.

Als organische Lösungsmittel sind beispielsweise geeignet : $CHCl_3$, Dichloräthan, Ligroin, Cyclohexan, $CH_2Cl_2$, $CS_2$, Nitrobenzol, Chlorbenzol und Dichlorbenzol.

Die so erhaltenen alkylsubstituierten Phenylpropylhalogenide können gegebenenfalls in weiteren Reaktionsstufen entsprechend dem folgenden Schema, in dem $R^1$, $R^2$, $R^3$, $R^4$, $R^5$ und X die oben genannten Bedeutungen haben,

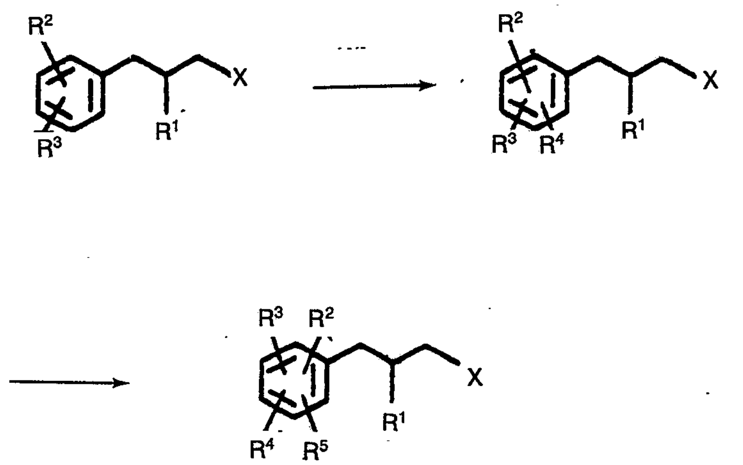

zu den entsprechenden Halogen-, Nitro- oder Acetylderivaten umgesetzt werden. Die Halogenierung erfolgt beispielsweise in üblicher Weise durch Umsetzung mit einem Halogen und Eisenpulver. Die Nitrierung erfolgt beispielsweise in üblicher Weise durch Umsetzung mit einer Mischung aus Schwefelsäure und Salpetersäure. Die Acetylierung erfolgt beispielsweise entsprechend G. Baddelay, J. Chem. Soc. *1956,* 4647.

Die folgenden Beispiele erläutern die Durchführung des Verfahrens.

Beispiel 1

Herstellung von 3-(4-tert.-Butylphenyl)-propylchlorid :

In eine Mischung von 62 Gewichtsteilen 3-Phenylpropylchlorid und 13 Gewichtsteilen 96 %iger (Gew.%) Schwefelsäure werden bei 10 °C 23 Gewichtsteile Isobutylen eingeleitet. Bei Raumtemperatur (20 °C) wird 3 Stunden weitergerührt. Das Rohprodukt wird in $CHCl_3$ gelöst, mit Eiswasser gründlich gewaschen, über $Na_2CO_3$ getrocknet und destilliert.

Man erhält 57 Gewichtsteile 3-(4-tert.-Butylphenyl)-propylchlorid, $Kp_{0,2}$ 108-110 °C.

Ausbeute 65 % (bez. auf 3-Phenylpropylchlorid).

Beispiel 2

Herstellung von 3-(4-Cyclopentylphenyl)-propylbromid :

Zu einer Mischung von 30 Gewichtsteilen 3-Phenylpropylbromid und 20 Gewichtsteilen 96 %iger Schwefelsäure werden 10 Gewichtsteile Cyclopenten bei 10 °C zugetropft. Bei Raumtemperatur (20 °C) wird 14 Stunden nachgerührt. Das Rohprodukt wird in $CHCl_3$ gelöst, mit Eiswasser gründlich gewaschen, über $Na_2CO_3$ getrocknet und destilliert. Man erhält 25 Gewichtsteile 3-(4-Cyclopentylphenyl)-propylbromid. $Kp_{0,5}$ 160-163 °C, Ausbeute 60 % (bezogen auf 3-Phenylpropylbromid).

Beispiel 3

Herstellung von 3-(4-Cyclohexylphenyl)-Propylbromid :

Zu einer Mischung von 40 Gewichtsteilen 3-Phenylpropylbromid und 20 Gewichtsteilen 96 %iger Schwefelsäure werden 17 Gewichtsteile Cyclohexen bei + 10 °C zugetropft. Bei Raumtemperatur wird 14 Stunden nechgerührt. Das Rohprodukt wird in $CHCl_3$ gelöst, mit Eiswasser gründlich gewaschen, über $Na_2CO_3$ getrocknet und destilliert. Man erhält 35 Gewichtsteile 3-(4-Cyclohexylphenyl)-propylbromid).

## Beispiel 4

Herstellung von 3-(4-Cyclohexylphenyl)-Propylchlorid :

Zu einer Mischung aus 31 Gewichtsteilen 3-Phenylpropylchlorid und 20 Gewichtsteilen 96 %iger Schwefelsäure werden 16,5 Gewichtsteile Cyclohexen bei + 10 °C zugetropft. Bei Raumtemperatur wird 14 Stud. nachgerührt. Das Rohprodukt wird in $CHCl_3$/Eiswasser gelöst, mit Eiswasser gründlich gewaschen, über $Na_2Co_3$ getrocknet und destilliert. Man erhält 27 Gewichtsteile 3-(4-Cyclohexylphenyl)-propylchlorid, $Kp_{0,2}$ 126-130 °C, Ausbeute 57 % (bezogen auf 3-Phenylpropylchlorid).

## Beispiel 5

Herstellung von 3-(4-Norbornylphenyl)-propylchlorid :

Zu einer Mischung aus 46 Gewichtsteilen 3-Phenylpropylchlorid und 30 Gewichtsteilen 96 %iger Schwefelsäure werden 28 Gewichtsteile geschmolzenes Norbornen bei 10 °C zugetropft. Bei Raumtemperatur wird 14 Std. nachgerührt. Das Rohprodukt wird in $CHCl_3$ gelöst, mit Eiswasser gründlich gewaschen, über $Na_2CO_3$ getrocknet und destilliert. Man erhält 61 Gewichtsteile 3-(4-Norbonyl-phenyl)-propylchlorid, $Kp_{0,1}$ 160-164 °C, Ausbeute 81 % (bezogen auf 3-Phenylpropylchlorid).

## Beispiel 6

Herstellung von 2-Methyl-3-(4-Norbornylphenyl)-propylchlorid :

Zu einer Mischung aus 42 Gewichtsteilen 2-Methyl-3-phenylpropylchlorid und 22 Gewichtsteilen 96 %iger Schwefelsäure werden 24 Gewichtsteile geschmolzenes Norbornen bei 10 °C zugetropft. Bei Raumtemperatur wird 14 Std. nachgerührt. Das Rohprodukt wird in $CHCl_3$ gelöst, mit Eiswasser gründlich gewaschen, über $Na_2CO_3$ getrocknet und destilliert. Man erhält 50 Gewichtsteile 2-Methyl-3-(4-Norbornylphenyl)-propylchlorid, $Kp_{0,1}$ 128-130 °C, Ausbeute 76 % (bezogen auf 2-Methyl-3-phenylpropylchlorid).

## Beispiel 7

Herstellung von 3-(4-Cyclohexylphenyl)-2-methylpropylchlorid :

Zu einer Mischung aus 67 Gewichtsteilen 2-Methyl-3-phenylpropylchlorid und 40 Gewichtsteilen 96 %iger Schwefelsäure werden 33 Gewichtsteile Cyclohexen bei 10 °C zugetropft. Bei Raumtemperatur wird 14 Std. nachgerührt. Das Rohprodukt wird in $CHCl_3$ gelöst, mit Eiswasser gründlich gewaschen, über $Na_2CO_3$ getrocknet und destilliert. Man erhält 70 Gewichtsteile 3-(4-Cyclohexylphenyl)-2-methylpropylchlorid, $Kp_{0,1}$ 130-130-2 °C, Ausbeute 70 % (bezogen auf 2-Methyl-3-phenylpropylchlorid).

## Beispiel 8

Herstellung von 3-(4-Heptylphenyl)-2-methylpropylchlorid :

Zu einer Mischung aus 42 Gewichtsteilen 2-Methyl-3-phenylpropylchlorid und 20 Gewichtsteilen 96 %iger Schwefelsäure werden 25 Gewichtsteile Hepten-1 bei 10 °C zugetropft. Bei Raumtemperatur wird 14 Std. nachgerührt. Das Rohprodukt wird in $CHCl_3$ gelöst, mis Eiswasser gewaschen, über $Na_2CO_3$ getrocknet und destilliert. Man erhält 50 Gewichtsteile 3-(4-Heptylphenyl)-2-methyl-propylchlorid, $Kp_{0,1}$ 114-7 °C, Ausbeute 75 % (bezogen auf 2-Methyl-3-phenylpropylchlorid).

## Beispiel 9

Herstellung von 3-p-(1-Chlormethyl-1-methyl-äthyl)-phenyl-2-methyl-propylchlorid :

Zu einer Mischung aus 17 Gewichtsteilen 2-Methyl-3-phenylpropylchlorid und 10 Gewichtsteilen 96 %iger Schwefelsäure werden 9 Gewichtsteile 2-Methylallylchlorid bei 10 °C zugetropft. Bei Raumtemperatur wird 14 Std. nachgerührt, das Rohprodukt wird in $CHCl_3$ gelöst, mit Wasser gewaschen, über $Na_2CO_3$ getrocknet und destilliert. Man erhält 14 Gewichtsteile 3-p-(1-Chlormethyl-1-methyl-äthyl)-

phenyl-2-methyl-propylchlorid, $Kp_{0,1}$ 129-131 °C, Ausbeute 54 % (bezogen auf 2-Methyl-3-phenyl-propylchlorid).

## Beispiel 10

Herstellung von 2-Methyl-3-(p-tert.-butylphenyl)-propylchlorid durch Alkylierung von 2-Methyl-3-phenylpropylchlorid nach verschiedenen Methoden :

A) Konzentrierte Schwefelsäure und Isobutylen :

In eine Mischung aus 84 Gewichtsteilen 2-Methyl-3-phenylpropylchlorid und 12,8 Gewichtsteilen 96 %iger Schwefelsäure werden bei + 10 °C 31 Gewichtsteile Isobutylen eingeleitet. Anschließend wird 3 Studen lang bei 10 °C weitergerührt. Danach läßt man die Mischung sich auf Raumtemperatur (20 °C) erwärmen, trennt die Schwefelsäurephase ab und wäscht die organische Phase mit 100 Volumenteilen 20 %iger Natronlauge. Zur besseren Phasentrennung werden 50 Volumenteile Aceton zugegeben. Danach wird die organische Phase abgetrennt und destilliert.

Man erhält

16 Gewichtsteile 2-Methyl-3-phenyl-propylchlorid, $Kp_{16} = 105$ °C

und

75 Gewichtsteile 2-Methyl-3-(p-tert.-butylphenyl)-propylchlorid, $Kp_{0,02} = 81$ °C.

Der Umsatz beträgt 81 %, die Ausbeute 82,5 % d. Th. (bez. auf umgesetztes 2-Methyl-3-phenylpropylchlorid).

B) Wasserfreies $FeCl_3$ und Isobutylen :

Bei 10 °C werden zu 84 Gewichtsteilen 2-Methyl-3-phenylpropylchlorid 20,3 Gewichtsteile feinteiliges wasserfreies Eisen-(III)-chlorid gegeben. Anschließend werden in dieses Gemisch bei 10 °C 31 Gewichtsteile Isobutylen eingeleitet, nach beendetem Einleiten wird 3 Stunden bei 10 °C nachgerührt. Dann läßt man die Mischung sich auf Raumtemperatur erwärmen, gibt unter Rühren und Kühlung 100 Volumenteile Wasser hinzu und filtriert vom Festprodukt ab.

Das Filtrat besteht aus einer wäßrigen und einer organischen Phase. Die organische Phase wird abgetrennt und destilliert.

Man erhält

5 Gewichtsteile 2-Methyl-3-phenyl-propylchlorid, $Kp_{16} = 105$ °C

und

74,5 Gewichtsteile 2-Methyl-3-(p-tert.-butylphenyl)-propylchlorid, $Kp_{0,02} = 80\text{-}81$ °C.

Der Umsatz beträgt 94 %, die Ausbeute 70 % d. Th. (bez. auf umgesetztes 2-Methyl-3-phenylpropylchlorid).

C) Bortrifluorid und tert.-Butylchlorid :

In eine Mischung aus 84 Gewichtsteilen 2-Methyl-3-phenyl-propylchlorid und 50 mL $BF_3.2H_2O$ wird bei Raumtemperatur 30 Minuten lang $BF_3$ eingeleitet. Anschließend werden 56 Gewichtsteile tert.-Butylchlorid bei Raumtemperatur zugetropft. Die Mischung wird 4 Std. lang zum Rückfluß erhitzt. Man läßt abkühlen, gibt das Rohprodukt in Wasser und extrahiert es mit Chloroform. Die organische Phase wird abgetrennt, mit Wasser gewaschen, über $Na_2CO_3$ getrocknet und destilliert. Man erhält 30 Gewichtsteile 2-Methyl-3-phenyl-propylchlorid, $Kp_{16} = 105$ °C und 45 Gewichtsteile 2-Methyl-3-(p-tert.-butylphenyl)-propylchlorid, $Kp_{0,02} = 80\text{-}81$ °C.

Der Umsatz beträgt 64 %, die Ausbeute 63 % bezogen auf umgesetztes 2-Methyl-3-phenylpropylchlorid.

D) Fluorwasserstoff und Isobutylen :

In eine Mischung aus 68 Gewichtsteilen 2-Methyl-3-phenylpropylchlorid und 3 Gewichtsteilen Fluorwasserstoff werden bei 0 °C 20 Gewichtsteile Isobuten eingeleitet. Anschließend wird 12 Std. lang bei Raumtemperatur gerührt. Das Rohprodukt wird mit Wasser gewaschen, über $Na_2CO_3$ getrocknet und destilliert. Man erhält 36 Gewichtsteile 2-Methyl-3-phenylpropylchlorid $Kp_{16} = 105$ °C und 32 Gewichtsteile 2-Methyl-3-(p-tert.-butylphenyl)-propylchlorid $Kp_{0,02} = 80\text{-}81$ °C.

Der Umsatz beträgt 47 %, die Ausbeute 75 % bezogen auf umgesetztes 2-Methyl-3-phenylpropylchlorid.

E) Eisen-(III)-chlorid und tert.-Butylchlorid :

In eine Mischung aus 100 Gewichtsteilen 2-Methyl-3-phenyl-propylchlorid und 8,1 Gewichtsteilen feinteiligem Eisen-(III)-chlorid (wasserfrei) werden bei 30 °C 61 Gewichtsteile tert.-Butylchlorid zugetropft. Bei Raumtemperatur wird 14 Std. nachgerührt. Das Rohprodukt wird in Eiswasser gegossen und mehrfach mit $CHCl_3$ extrahiert, die Chloroformlösung abgetrennt, mit $Na_2CO_3$ getrocknet und destilliert.

Man erhält

42 Gewichtsteile 2-Methyl-3-phenyl-propylchlorid $Kp_{16}$ 105 °C und 55 Gewichtsteile 2-Methyl-3-(p-tert.-butylphenyl)-propylchlorid, $Kp_{0,02} = 80\text{-}82$ °C.

Der Umsatz beträgt 58 %, die Ausbeute 71 %, bezogen auf umgesetztes 2-Methyl-3-phenyl-propylchlorid.

Beispiel 11

Herstellung von 3-(5-tert.-Butyl-2-methoxy-phenyl)-2-methyl-propylchlorid :

Zu einer Mischung aus :

234 Gewichtsteilen 3-(2-Methoxy-phenyl)-2-methyl-propylchlorid und 19,4 Gewichtsteilen Eisen-(III)-chlorid (wasserfrei) werden bei 30 °C 122 Gewichtsteile tert.-Butylchlorid zugetropft. Man rührt 8 Std. bei 50 °C und 14 Std. bei Raumtemperatur. Das Rohprodukt wird in Eiswasser gegossen, in $CHCl_3$ gelöst, mehrfach mit Wasser gewaschen, über $Na_2CO_3$ getrocknet, das Lösungsmittel verdampft und der Rückstand destilliert.

Man erhält 110 Gewichsteile 3-(5-tert.-Butyl-2-methoxy-phenyl)-2-methyl-propylchlorid, $Kp_{0,1}$ = 105-108 °C

Beispiel 12

Herstellung von 3-(5-tert.-Butyl-2-methyl-phenyl)-2-methyl-propylchlorid und von 3-(4-tert.-Butyl-2-methyl-phenyl)-2-methyl-propylchlorid :

In eine Mischung aus 200 Gewichtsteilen 3-(2-Methyl-phenyl)-2-methyl-propylchlorid und 98 Gewichtsteilen 96 %iger Schwefelsäure werden bei + 10 °C 62 Gewichtsteile Isobutylen eingeleitet. Anschließend wird 3 Std. bei 10 °C nachgerührt. Man läßt die Mischung sich auf Raumtemperatur erwärmen, trennt die Schwefelsäurephase ab und wäscht die organische Phase mit 100 Volumenteilen 20 %iger Natronlauge. Zur besseren Phasentrennung werden 50 Volumenteile Aceton zugegeben. Danach wird die organische Phase abgetrennt und destilliert.

Man erhält ein Gemisch bestehend aus 3-(5-tert.-Butyl-2-methyl-phenyl)-2-methyl-propylchlorid und 3-(4-tert.-Butyl-2-methyl-phenyl)-2-methyl-propyl-chlorid. $Kp_{0,1}$ = 106 °C.

Beispiel 13

Herstellung von 3-[p-(1-Methyl-1-äthyl-propyl)-phenyl]-2-methyl-propylchlorid :

Zu einer Mischung aus 510 Gewichtsteilen 2-Methyl-3-phenyl-propylchlorid une 49 Gewichtsteilen Eisen-(III)-chlorid (wasserfrei) werden bei 30 °C 365 Gewichtsteile 1-Methyl-1-äthyl-propylchlorid zugetropft. Man rührt 8 Std. bei 50 °C und 14 Std. bei Raumtemperatur (20 °C). Das Rohprodukt wird in Eiswasser gegossen, in $CHCl_3$ gelöst, mehrfach mit Wasser gewaschen, über $Na_2CO_3$ getrocknet, das Lösungsmittel verdampft und der Rückstand destilliert.

Man erhält 420 Gewichtsteile 3-[p-(1-methyl-1-äthyl-propyl)-phenyl]-2-methyl-propylchlorid. $Kp_{0,1}$ = 125 °C.

Beispiel 14

Herstellung von 3-(p-tert.-Butyl-o-brom-phenyl)-2-methyl-propylchlorid :

Zu einer Mischung aus 23 Gewichtsteilen 3-(p-tert.-Butyl-phenyl)-2-methyl-propylchlorid und 0,5 Gewichtsteilen Eisenpulver werden 16 Gewichtsteile Brom zugetropft. Die Reaktionstemperatur steigt dabei auf 35 °C an. Man rührt 17 Std. nach, löst in Chloroform, wäscht die Lösung mit Wasser, mit wäßriger $NaHCO_3$-Lösung und wieder mit Wasser, trocknet über $Na_2SO_4$, verdampft das Lösungsmittel und destilliert den Rückstand.

Man erhält 20 Gewichtsteile 3-(p-tert.-Butyl-o-brom-phenyl)-2-methyl-propylchlorid). $Kp_{0,1}$ 111-115 °C.

Beispiel 15

Herstellung von 3-(p-tert.-Butyl-o-chlorphenyl)-2-methyl-propylchlorid und 3-(p-tert.-Butyl-m-chlorphenyl)-2-methyl-propylchlorid :

Zu einer Mischung aus 320 Gewichtsteilen 3-(p-tert.-Butyl-phenyl)-2-methyl-propylchlorid und 2 Gewichtsteilen Eisenpulver werden bei 30-35 °C 101 Gewichtsteile Chlor eingegast. Man rührt 1 Std. nach und gießt das Rohprodukt in Eiswasser. Die organische Phase wird mit Wasser, wäßriger $NaCHO_3$-Lösung und wieder mit Wasser gewaschen, über $Na_2SO_4$ getrocknet, das Lösungsmittel verdampft und der Rückstand destilliert. Man erhält 300 Gewichtsteile einer Mischung aus 3-(p-tert.-Butyl-o-chlorphenyl)-2-methyl-propylchlorid und 3-(p-tert.-Butyl-m-chlor-phenyl)-2-methyl-propylchlorid und 3-(p-tert.-Butyl-m-chlor-phenyl)-2-methyl-propylchlorid. $Kp_{0,1}$ = 106-111 °C.

Beispiel 16

Hestellung von 3-(p-tert.-Amyl-o-chlor-phenyl)-2-methyl-propylchlorid und 3-(p-tert.-Amyl-m-chlor-phenyl)-2-methyl-propylchlorid :

Zu einer Mischung aus 177 Gewichtsteilen 3-(p-tert.-Amyl-phenyl)-2-methyl-propylchlorid und 1 Gewichtsteil Eisenpulver werden bei 30-35 °C 63 Gewichtsteile Chlor eingegast. Man rührt 14 Std. bei Raumtemperatur nach, löst in Chloroform, wäscht mit Wasser, 10 %iger Natronlauge und wieder mit Wasser, trocknet über $Na_2SO_4$, verdampft das Lösungsmittel und destilliert den Rückstand.

Man erhält 110 Gewichtsteile einer Mischung aus 3-(p-tert.-Amyl-o-chlor-phenyl)-2-methyl-propylchlorid und 3-(p-tert.-Amyl-m-chlor-phenyl)-2-methyl-propylchlorid. $Kp_{0,1}$ = 118-120 °C.

Beispiel 17

Herstellung von 3-(p-tert.-Butyl-o-nitro-phenyl)-2-methyl-propylchlorid :

Zu 448 Gewichtsteilen 3-(p-tert.-Butylphenyl)-2-methyl-propylchlorid wird unter Eiskühlung 440 Gewichtsteile Nitriersäure, bestehend aus 200 Gewichtsteilen konz. Salpetersäure und 240 Gewichtsteilen konz. Schwefelsäure zugetropft. Nach 3 Std. Nachrühren bei Raumtemperatur wird das Rohprodukt auf Eis gegossen, mit Diäthyläther extrahiert, mit Wasser, wäßriger $NaHCO_3$-Lösung und wieder Wasser gewaschen, über $Na_2SO_4$ getrocknet, verdampft das Lösungsmittel und destilliert den Rückstand.

Man erhält 413 Gewichtsteile 3-(p-tert.-Butyl-o-nitro-phenyl)-2-methyl-propylchlorid. $Kp_{0,1}$ = 138-140 °C.

Beispiel 18

Herstellung von 2-Methyl-3-(o-acetyl-p-tert.-butyl-phenyl)-propylchlorid :

Zu einer Lösung von 60 g 3-(p-tert.-Butyl-phenyl)-2-methyl-propylchlorid in 50 ml Methylenchlorid wird bei 20 °C eine Lösung von 63 g Acetylchlorid und 53 g Aluminiumchlorid in 300 ml Methylenchlorid zugetropft. Nach 10 Minuten Nachrühren bei Raumtemperatur wird die Mischung in Eiswasser gegossen, mit Methylenchlorid extrahiert, mehrfach mit Wasser gewaschen, über $Na_2SO_4$ getrocknet, man verdampft das Lösungsmittel und destilliert den Rückstand.

Man erhält 30 g 2-Methyl-3-(o-acetyl-p-tert.-butyl-phenyl)-propylchlorid. $Kp_{0,1}$ = 134 °C.

Beispiel 19

Darstellung von 3-(p-tert.-Butyl-o-chlor-phenyl)-2-methyl-propylbromid (1) und 3-(p-tert.-Butyl-m-chlor-phenyl)-2-methyl-propylbromid (2).

Die Herstellung erfolgt analog Beispiel 15 :
$Kp_{0,2}$ = 120-3°, enthält 70 % (1) und 30 % (2).

Beispiel 20

3-[p-(1,1-Dimethyl-butyl)-phenyl]-2-methyl-propylchlorid.
Die Herstellung erfolgt analog Beispiel 10E. $Kp_{0,1}$ = 116-118 °C.

Beispiel 21

3-[p-(1-Äthyl-1-methyl-propyl)-o-phenyl]-2-methyl-propylchlorid und 3-[p-(1-Äthyl-1-methyl-propyl)-m-chlor-phenyl]-2-methyl-propylchlorid.
Die Herstellung erfolgt analog Beispiel 10E. $Kp_{0,1}$ = 135-138 °C.

Beispiel 22

3-(p-Methoxy-o-tert.-amyl-phenyl)-2-methyl-propylchlorid und 3-(p-Methoxy-m-tert.-amyl-phenyl)-2-methyl-propylchlorid.
Die Herstellung erfolgt analog Beispiel 10E. $Kp_{0,1}$ = 123-125 °C.

Beispiel 23

2-(p-tert.-Amyl-phenyl)-propylchlorid.
Die Herstellung erfolgt analog Beispiel 10E. $Kp_{0,1}$ = 114-115 °C.

**Ansprüche** für die Vertragsstaaten : BE, CH, DE, FR, GB, IT, LU, NL, SE

1. Verfahren zur Herstellung von Phenylpropylhalogeniden der allgemeinen Formel

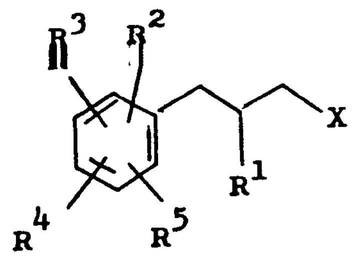

(I)

in der $R^1$ Wasserstoff oder einen aliphatischen Rest mit 1 bis 10 Kohlenstoffatomen, $R^2$ Wasserstoff, einen Alkylrest mit 1 bis 3 Kohlenstoffatomen, Fluor oder einen Methoxyrest, $R^3$ einen aliphatischen Rest mit 2 bis 20 Kohlenstoffatomen, einen cyclo- oder bicycloaliphatischen Rest mit 5 bis 7 Kohlenstoffatomen oder einen Halogenalkylrest mit 3 bis 10 Kohlenstoffatomen, $R^4$ und $R^5$ jeweils unabhängig voneinander Wasserstoff, Chlor, Brom, Nitro oder Acetyl und X Chlor oder Brom bedeuten, dadurch gekennzeichnet, daß man ein Phenylpropylhalogenid der Formel

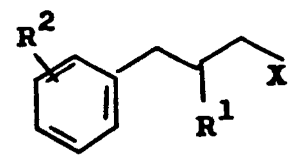

(II)

worin $R^1$, $R^2$ und X die oben angegebenen Bedeutungen haben in Gegenwart eines sauren Katalysators mit einem Alkohol der Formel $R^3OH$ oder einem Alkylhalogenid der Formel $R^3Hal$, wobei Hal ein Halogenatom bedeutet, oder einem der Verbindung $R^3H$ entsprechendem Olefin umsetzt, wobei $R^3$ die oben genannten Bedeutungen hat und gegebenenfalls das so erhaltene Umsetzungsprodukt in einer an sich bekannten Weise zu dem entsprechenden Halogenderivat, Nitroderivat oder Acetylderivat umsetzt.

2. Phenylpropylhalogenide der allgemeinen Formel

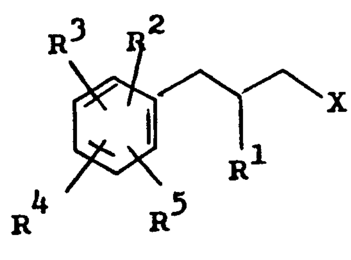

(III)

in der $R^1$ Wasserstoff oder einen aliphatischen Rest mit 1 bis 10 Kohlenstoffatomen, $R^2$ Wasserstoff, einen Alkylrest mit 1 bis 3 Kohlenstoffatomen, Fluor oder einen Methoxyrest, $R^3$ einen aliphatischen Rest mit 2 bis 20 Kohlenstoffatomen, einen cyclo- oder bicycloaliphatischen Rest mit 5 bis 7 Kohlenstoffatomen oder einen Halogenalkylrest mit 3 bis 10 Kohlenstoffatomen, $R^4$ une $R^5$ jeweils unabhängig voneinander Wasserstoff, Chlor, Brom, Nitro oder Acetyl und X Chlor oder Brom bedeutet, mit der Maßgabe, daß $R^3$ einen Halogenalkylrest mit 3 bis 10 Kohlenstoffatomen bedeutet, wenn $R^2$, $R^4$ und $R^5$ gleichzeitig Wasserstoff bedeuten.

3. Verfahren gemäß Anspruch 1, dadurch gekennzeichnet, daß man die Umsetzung mit einem der Formel $R^3H$ entsprechenden Olefin in Gegenwart von $H_2SO_4$, HF oder $FeCl_3$ durchführt, wobei $R^3$ die in Anspruch 1 genannten Bedeutungen hat.

4. Verfahren gemäß Anspruch 1, dadurch gekennzeichnet, daß man die Umsetzung mit einem tertiären Alkylchlorid in Gegenwart von $FeCl_3$ durchführt.

5. Verfahren gemäß Anspruch 1, dadurch gekennzeichnet, daß man 2-Methyl-3-(phenyl)-propylchlorid mit Isobutylen in Gegenwart von konzentrierter Schwefelsäure umsetzt.

6. 3-(p-1-Chlormethyl-1-methyl-äthyl-phenyl)-2-methyl-propylchlorid.

7. 3-(p-tert.-Butyl-o-chlor-phenyl)-2-methyl-propylchlorid.

8. 3-(p-tert.-Butyl-o-chlor-phenyl)-2-methyl-propylbromid.

9. Verwendung von Phenylpropylhalogeniden der allgemeinen Formel

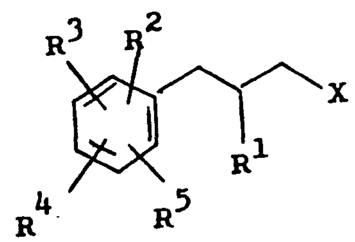

(III),

in der $R^1$ Wasserstoff oder einen aliphatischen Rest mit 1 bis 10 Kohlenstoffatomen, $R^2$ Wasserstoff, einen Alkylrest mit 1 bis 3 Kohlenstoffatomen, Fluor oder einen Methoxyrest, $R^3$ einen aliphatischen Rest mit 2 bis 20 Kohlenstoffatomen, einen cyclo- oder bicycloaliphatischen Rest mit 5 bis 7 Kohlenstoffatomen oder einen Halogenalkylrest mit 3 bis 10 Kohlenstoffatomen, $R^4$ und $R^5$ jeweils unabhängig voneinander Wasserstoff, Chlor, Brom, Nitro oder Acetyl und X Chlor oder Brom bedeutet, mit der Maßgabe, daß $R^3$ einen Halogenalkylrest mit 3 bis 10 Kohlenstoffatomen bedeutet, wenn $R^2$, $R^4$ und $R^5$ gleichzeitig Wasserstoff bedeuten, zur Umsetzung mit einem gegebenenfalls substituierten Morpholin oder Piperidin.

**Ansprüche** für den Vertragsstaat : AT

1. Verfahren zur Herstellung von Phenylpropylhalogeniden der allgemeinen Formel

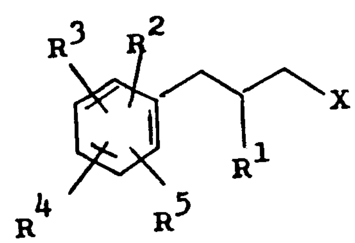

(I)

in der $R^1$ Wasserstoff oder einen aliphatischen Rest mit 1 bis 10 Kohlenstoffatomen, $R^2$ Wasserstoff, einen Alkylrest mit 1 bis 3 Kohlenstoffatomen, Fluor oder einen Methoxyrest, $R^3$ einen aliphatischen Rest mit 2 bis 20 Kohlenstoffatomen, einen cyclo- oder bicycloaliphatischen Rest mit 5 bis 7 Kohlenstoffatomen oder einen Halogenalkylrest mit 3 bis 10 Kohlenstoffatomen, $R^4$ und $R^5$ jeweils unabhängig voneinander Wasserstoff, Chlor, Brom, Nitro oder Acetyl und X Chlor oder Brom bedeuten, dadurch gekennzeichnet, daß man ein Phenylpropylhalogenid der Formel

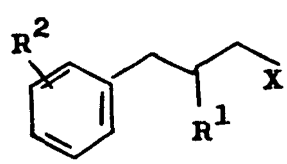

(II)

worin $R^1$, $R^2$ und X die oben angegebenen Bedeutungen haben in Gegenwart eines sauren Katalysators mit einem Alkohol der Formel $R^3OH$ oder einem Alkylhalogenid der Formel $R^3Hal$, wobei Hal ein Halogenatom bedeutet, oder einem der Verbindung $R^3H$ entsprechendem Olefin umsetzt, wobei $R^3$ die oben genannten Bedeutungen hat und gegebenenfalls das so erhaltene Umsetzungsprodukt in einer an sich bekannten Weise zu dem entsprechenden Halogenderivat, Nitroderivat oder Acetylderivat umsetzt.

2. Verfahren gemäß Anspruch 1, dadurch gekennzeichnet, daß man die Umsetzung mit einem der Formel $R^3H$ entsprechenden Olefin in Gegenwart von $H_2SO_4$, HF oder $FeCl_3$ durchführt, wobei $R^3$ die in Anspruch 1 genannten Bedeutungen hat.

3. Verfahren gemäß Anspruch 1, dadurch gekennzeichnet, daß man die Umsetzung mit einem tertiären Alkylchlorid in Gegenwart von $FeCl_3$ durchführt.

4. Verfahren gemäß Anspruch 1, dadurch gekennzeichnet, daß man 2-Methyl-3-(phenyl)-propylchlorid mit Isobutylen in Gegenwart von konzentrierter Schwefelsäure umsetzt.

5. Verwendung von Phenylpropylhalogeniden der allgemeinen Formel

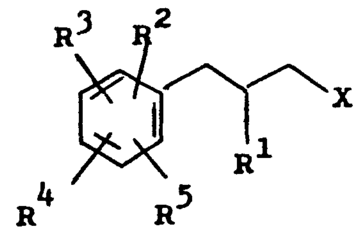

(III),

in der $R^1$ Wasserstoff oder einen aliphatischen Rest mit 1 bis 10 Kohlenstoffatomen, $R^2$ Wasserstoff, einen Alkylrest mit 1 bis 3 Kohlenstoffatomen, Fluor oder einen Methoxyrest. $R^3$ einen aliphatischen Rest mit 2 bis 20 Kohlenstoffatomen, einen cyclo- oder bicycloaliphatischen Rest mit 5 bis 7 Kohlenstoffatomen oder einen Halogenalkylrest mit 3 bis 10 Kohlenstoffatomen, $R^4$ und $R^5$ jeweils unabhängig voneinander Wasserstoff, Chlor, Brom, Nitro oder Acetyl und X Chlor oder Brom bedeutet, der Maßgabe, daß $R^3$ einen Halogenalkylrest mit 3 bis 10 Kohlenstoffatomen bedeutet, wenn $R^2$, $R^4$ und $R^5$ gleichzeitig Wasserstoff bedeuten, zur Umsetzung mit einem gegebenenfalls substituierten Morpholin oder Piperidin.

**Claims** for the contracting states : BE, CH, DE, FR, GB, IT, LU, NL, SE

1. A process for the preparation of a phenylpropyl halide of the general formula

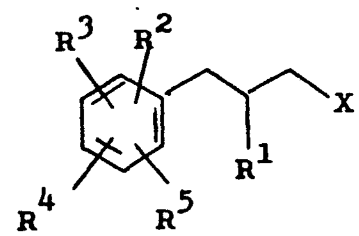

(I)

where $R^1$ is hydrogen or an aliphatic radical of 1 to 10 carbon atoms, $R^2$ is hydrogen, alkyl of 1 to 3 carbon atoms, fluorine or methoxy, $R^3$ is an aliphatic radical of 2 to 20 carbon atoms, a cycloaliphatic or bicycloaliphatic radical of 5 to 7 carbon atoms or haloalkyl of 3 to 10 carbon atoms, $R^4$ and $R^5$ independently of one another are each hydrogen, chlorine, bromine, nitro or acetyl and X is chlorine or bromine, characterized in that a phenylpropyl halide of the formula

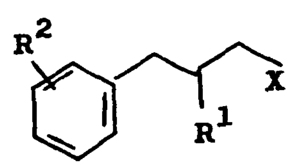

(II)

where $R^1$, $R^2$ and X have the above meanings, is reacted, in the presence of an acid catalyst, with an alcohol of the formula $R^3OH$ or an alkyl halide of the formula $R^3Hal$, where Hal is halogen, or with an olefin corresponding to the compound $R^3H$, where $R^3$ has the above meanings, and, where required, the reaction product thus obtained is converted in a conventional manner into the corresponding halogen derivative, nitro derivative or acetyl derivative.

2. A phenylpropyl halide of the general formula

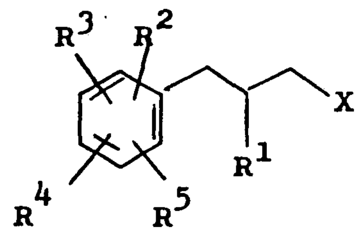

(III)

where $R^1$ is hydrogen or an aliphatic radical of 1 to 10 carbon atoms, $R^2$ is hydrogen, alkyl of 1 to 3 carbon atoms, fluorine or methoxy, $R^3$ is an aliphatic radical of 2 to 20 carbons atoms, a cycloaliphatic or bicycloaliphatic radical of 5 to 7 carbon atoms or haloalkyl of 3 to 10 carbon atoms, $R^4$ and $R^5$ independently of one another are each hydrogen, chlorine, bromine, nitro or acetyl, and X is chlorine or bromine, with the proviso that $R^3$ is haloalkyl of 3 to 10 carbon atoms if $R^2$, $R^4$ and $R^5$ are simultaneously hydrogen.

3. A process as claimed in claim 1, characterized in that the reaction is carried out with an olefin corresponding to the compound $R^3H$, where $R^3$ has the meanings given in claim 1, in the presence of $H_2SO_4$, HF or $FeCl_3$.

4. A process as claimed in claim 1, characterized in that the reaction is carried out with a tertiary alkyl chloride in the presence of $FeCl_3$.

5. A process as claimed in claim 1, characterized in that 2-methyl-3-phenyl-propyl chloride is reacted with isobutylene in the presence of concentrated sulfuric acid.

6. 3-(p-1-Chloromethyl-1-methyl-ethyl-phenyl)-2-methyl-propyl chloride.

7. 3-(p-tert.-Butyl-o-chloro-phenyl)-2-methyl-propyl chloride.

8. 3-(p-tert.-Butyl-o-chloro-phenyl)-2-methyl-propyl bromide.

9. Use of a phenylpropyl halide of the general formula

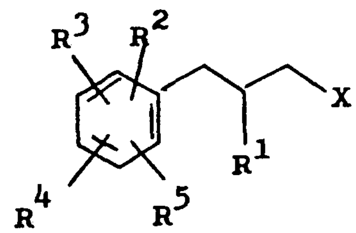

(III)

where $R^1$ is hydrogen or an aliphatic radical of 1 to 10 carbon atoms, $R^2$ is hydrogen, alkyl of 1 to 3 carbon atoms, fluorine or methoxy, $R^3$ is an aliphatic radical of 2 to 20 carbon atoms, a cycloaliphatic or bicycloaliphatic radical of 5 to 7 carbon atoms or haloalkyl of 3 to 10 carbon atoms, $R^4$ and $R^5$ indepently of one another are each hydrogen, chlorine, bromine, nitro or acetyl, and X is chlorine or bromine, with the proviso that $R^3$ is haloalkyl of 3 to 10 carbon atoms if $R^2$, $R^4$ and $R^5$ are simultaneously hydrogen, for reaction with an unsubstituted or substituted morpholine or piperidine.

**Claims** for the contracting state : AT

1. A process for the preparation of a phenylpropyl halide of the general formula

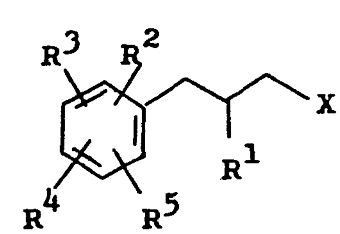

(I)

where $R^1$ is hydrogen or an aliphatic radical of a to 10 carbon atoms, $R^2$ is hydrogen, alkyl of 1 to

3 carbon atoms, fluorine or methoxy, $R^3$ is an aliphatic radical of 2 to 20 carbons atoms, a cycloaliphatic or bicycloaliphatic radical of 5 to 7 carbon atoms or haloalkyl of 3 to 10 carbon atoms, $R^4$ and $R^5$ independently of one another are each hydrogen, chlorine, bromine, nitro or acetyl and X is chlorine or bromine, characterized in that a phenylpropyl halide of the formula

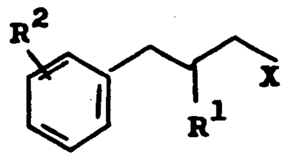

(II)

where $R^1$, $R^2$ and X have the above meanings, is reacted, in the presence of an acid catalyst, with an alcohol of the formula $R^3OH$ or an alkyl halide of the formula $R^3Hal$, where Hal is halogen, or with an olefin corresponding to the compound $R^3Hal$, where $R^3$ has the above meanings, and, where required, the reaction product thus obtained is converted in a conventional manner into the corresponding halogen derivative, nitro derivative or acetyl derivative.

2. A process as claimed in claim 1, characterized in that the reaction is carried out with an olefin corresponding to the compound $R^3H$, where $R^3$ has the meanings given in claim 1, in the presence of $H_2SO_4$, HF or $FeCl_3$.

3. A process as claimed in claim 1, characterized in that the reaction is carried out with a tertiary alkyl chloride in the presence of $FeCl_3$.

4. A process as claimed in claim 1, characterized in that 2-methyl-3-phenyl-propyl chloride is reacted with isobutylene in the presence of concentrated sulfuric acid.

5. Use of a phenylpropyl halide of the general formula

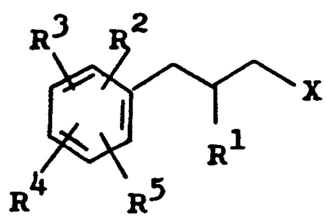

(III)

where $R^1$ is hydrogen or an aliphatic radical of 1 to 10 carbon atoms, $R^2$ is hydrogen, alkyl of 1 to 3 carbon atoms, fluorine or methoxy, $R^3$ is an aliphatic radical of 2 to 20 carbon atoms, a cycloaliphatic or bicycloaliphatic radical of 5 to 7 carbon atoms or haloalkyl of 3 to 10 carbon atoms, $R^4$ and $R^5$ independently of one another are each hydrogen, chlorine, bromine, nitro or acetyl, and X is chlorine or bromine, with the proviso that $R^3$ is haloalkyl of 3 to 10 carbon atoms if $R^2$, $R^4$ and $R^5$ are simultaneously hydrogen, for reaction with an unsubstituted or substituted morpholine or piperidine.

**Revendications** pour les Etats contractants : BE, CH, DE, FR, GB, IT, LU, NL, SE

1. Procédé de préparation d'halogénures de phénylpropyle de la formule générale

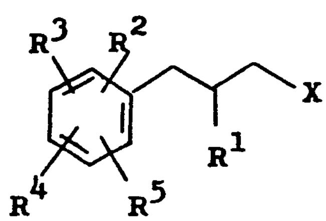

(I)

dans laquelle $R^1$ représente hydrogène ou un reste aliphatique en $C_1$ à $C_{10}$,

$R^2$ hydrogène, un reste alkyle en $C_1$ à $C_3$ fluor ou un reste méthoxy,

$R^3$ un reste aliphatique en $C_2$ à $C_{20}$ un reste cyclo- ou bicycloaliphatique en $C_5$ à $C_7$ ou un reste halogénoalkyle en $C_3$ à $C_{10}$,

$R^4$ et $R^5$ chacun indépendamment l'un de l'autre, hydrogène, chlore, brome, nitro ou acétyle et X chlore ou brome, caractérisé par le fait que l'on fait réagir un halogénure de phénylpropyle de formule

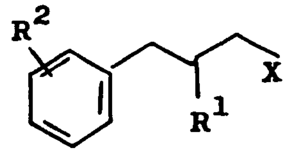

(II)

dans laquelle $R^1$, $R^2$ et X ont les significations données plus haut, en présence d'un catalyseur acide, avec un alcool de formule $R^3OH$ ou un halogénure d'alkyle de formule $R^3Hal$, Hal représentant un atome d'halogène, ou une oléfine correspondant au composé $R^3H$, $R^3$ ayant la signification indiquée plus haut, et que l'on transforme éventuellement, de manière connue en soi, le produit de réaction ainsi obtenu en le dérivé halogène, dérivé nitro ou dérivé acétyle correspondant.

2. Halogénure de phénylpropyle de formule générale

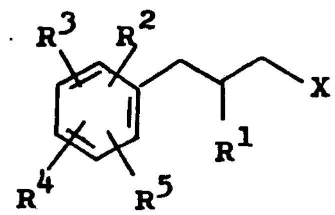

(III)

dans laquelle $R^1$ représente hydrogène ou un reste aliphatique en $C_1$ à $C_{10}$, $R^2$ hydrogène, un reste alkyle en $C_1$ à $C_3$, fluor ou un reste méthoxy, $R^3$ un reste aliphatique en $C_2$ à $C_{20}$, un reste cyclo- ou bicycloaliphatique en $C_5$ à $C_7$ ou un reste halogénoalkyle en $C_3$ à $C_{10}$, $R^4$ et $R^5$ chacun indépendamment l'un de l'autre, hydrogène, chlore, brome, nitro ou acétyle et X chlore ou brome sous réserve que $R^3$ représente un reste halogénalkyle en $C_3$ à $C_{10}$ lorsque $R^2$, $R^4$ et $R^5$ représentent simultanément l'hydrogène.

3. Procédé selon la revendication 1, caractérisé par le fait que l'on effectue la réaction avec une oléfine correspondant à la formule $R^3H$, en présence de $H_2SO_4$, HF ou $FeCl_3$, $R^3$ ayant la signification indiquée dans la revendication 1.

4. Procédé selon la revendication 1, caractérisé par le fait que l'on effectue la réaction avec un chlorure d'alkyle tertiaire, en présence de $FeCl_3$.

5. Procédé selon la revendication 1, caractérisé par le fait que l'on fait réagir du chlorure de 2-méthyl-3-phénylpropyle avec de l'isobutylène en présence d'acide sulfurique concentré.

6. Chlorure de 3-(p-1-chlorométhyl-1-méthyl-éthyl-phényl)-2-méthyl-propyle.

7. Chlorure de 3-(p-tert.-butyl-o-chloro-phényl)-2-méthyl-propyle.

8. Bromure de 3-(p-tert.-butyl-o-chloro-phényl)-2-méthyl-propyle.

9. Utilisation, pour réaction avec une morpholine ou pipéridine, éventuellement substituée, d'halogénures de phénylpropyle de formule générale III

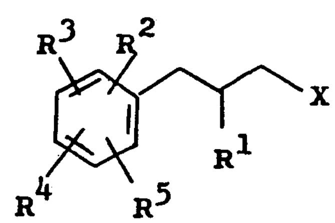

(III)

dans laquelle $R^1$ représente hydrogène ou un reste aliphatique en $C_1$ à $C_{10}$, $R^2$ hydrogène, un reste alkyle en $C_1$ à $C_3$, fluor ou un reste méthoxy, $R^3$ un reste aliphatique en $C_2$ à $C_{20}$, un reste cyclo- ou bicycloaliphatique en $C_5$ à $C_7$ ou un reste halogénoalkyle en $C_3$ à $C_{10}$, $R^4$ et $R^5$ chacun indépendamment

l'un de l'autre, hydrogène, chlore, brome, nitro ou acétyle et X chlore ou brome, sous réserve que $R^3$ représente un reste halogénalkyle en $C_3$ à $C_{10}$ lorsque $R^2$, $R^4$ et $R^5$ représentent simultanément l'hydrogène.

**Revendications** pour l'Etat contractant : AT

1. Procédé de préparation d'halogénures de phénylpropyle de la formule générale

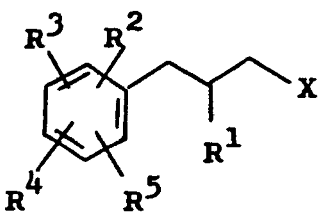

(I)

dans laquelle $R^1$ représente hydrogène ou un reste aliphatique en $C_1$ à $C_{10}$, $R^2$ hydrogène, un reste alkyle en $C_1$ à $C_3$, fluor ou un reste méthoxy, $R^3$ un reste aliphatique en $C_2$ à $C_{20}$, un reste cyclo- ou bicycloaliphatique en $C_5$ à $C_7$ ou un reste halogénoalkyle en $C_3$ à $C_{10}$, $R^4$ et $R^5$ chacun indépendamment l'un de l'autre, hydrogène, chloré, brome, nitro ou acétyle et X chlore ou brome, caractérisé par le fait que l'on fait réagir un halogénure de phénylpropyle de formule

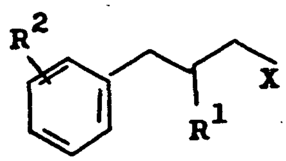

(II)

dans laquelle $R^1$, $R^2$ et X ont les significations données plus haut, en présence d'un catalyseur acide, avec un alcool de formule $R^3OH$ ou un halogénure d'alkyle de formule $R^3Hal$, Hal représentant un atome d'halogène, ou une oléfine correspondant au composé $R^3H$, $R^3$ ayant la signification indiquée plus haut et que l'on transforme éventuellement, de manière connue en soi, le produit de réaction ainsi obtenu en le dérivé halogène, dérivé nitro ou dérivé acétyle correspondant.

2. Procédé selon la revendication 1, caractérisé par le fait que l'on effectue la réaction avec une oléfine correspondant à la formule $R^3H$, en présence de $H_2SO_4$, HF ou $FeCl_3$, $R^3$ ayant la signification indiquée dans la revendication 1.

3. Procédé selon la revendication 1, caractérisé par le fait que l'on effectue la réaction avec un chlorure d'alkyle tertiaire, en présence de $FeCl_3$.

4. Procédé selon la revendication 1, caractérisé par le fait que l'on fait réagir du chlorure de 2-méthyl-3-phénylpropyle avec de l'isobutylène en présence d'acide sulfurique concentré.

5. Utilisation, pour réaction avec une morpholine ou pipéridine, éventuellement substituée, d'halogénures de phénylpropyle de formule générale III

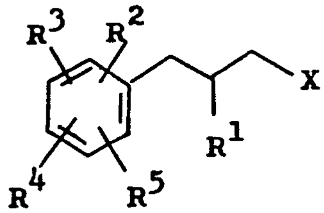

(III)

dans laquelle $R^1$ représente hydrogène ou un reste aliphatique en $C_1$ à $C_{10}$, $R^2$ hydrogène, un reste alkyle en $C_1$ à $C_3$, fluor ou un reste méthoxy, $R^3$ un reste aliphatique en $C_2$ à $C_{20}$, un reste cyclo- ou bicycloaliphatique en $C_5$ à $C_7$ ou un reste halogénoalkyle en $C_3$ à $C_{10}$, $R^4$ et $R^5$ chacun indépendamment l'un de l'autre, hydrogène, chlore, brome, nitro ou acétyle et X chlore ou brome, sous réserve que $R^3$ représente un reste halogénoalkyle en $C_3$ à $C_{10}$ lorsque $R^2$, $R^4$ et $R^5$ représentent simultanément l'hydrogène.